(19) Europäisches Patentamt
European Patent Office
Office européen des brevets

(11) EP 3 561 535 A1

(12) EUROPÄISCHE PATENTANMELDUNG

(43) Veröffentlichungstag:
30.10.2019 Patentblatt 2019/44

(51) Int Cl.:
G01R 33/48 (2006.01)     G01R 33/483 (2006.01)
G01R 33/561 (2006.01)

(21) Anmeldenummer: 18169552.9

(22) Anmeldetag: 26.04.2018

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME
Benannte Validierungsstaaten:
KH MA MD TN

(71) Anmelder: Siemens Healthcare GmbH
91052 Erlangen (DE)

(72) Erfinder:
• Carinci, Flavio
  91052 Erlangen (DE)
• Zeller, Mario
  91054 Erlangen (DE)

Bemerkungen:
Geänderte Patentansprüche gemäss Regel 137(2)
EPÜ.

(54) SIMULTANE MEHRSCHICHT-MAGNETRESONANZBILDGEBUNG MIT VARIABLER DICHTE DER K-RAUM-ABTASTUNG

(57) Die Erfindung betrifft ein Verfahren zur Aufnahme eines Magnetresonanzdatensatzes mit mehreren Spulen (6, 7, 8, 9), wobei die Messsignale(16, 35, 36, 43) des Magnetresonanzdatensatzes Messsignale (16, 35, 36, 43) aus wenigstens zwei Schichten (40, 41) enthalten, wobei die Messsignale (16, 35, 36, 43) segmentiert aufgenommen werden, dadurch gekennzeichnet, dass die Messsignale (16, 35, 36, 43) in einem ersten Bereich (34) des k-Raums mit einer ersten Abtastdichte ($\Delta k_{y1}$) und in einem zweiten Bereich (37) des k-Raums mit einer zweiten Abtastdichte ($\Delta k_{y2}$) aufgenommen werden.

Die Erfindung betrifft ferner ein Computerprogrammprodukt.

Die Erfindung betrifft ferner einen Datenträger.

Die Erfindung betrifft ferner eine Magnetresonanzanlage, mit der das vorgenannte Verfahren ausführbar ist.

FIG 3

**Beschreibung**

Die Erfindung betrifft ein Verfahren zur Aufnahme eines Magnetresonanzdatensatzes mit mehreren Spulen, wobei die Messsignale des Magnetresonanzdatensatzes Messsignale aus wenigstens zwei Schichten enthalten, wobei die Messsignale segmentiert aufgenommen werden.

Bei der Magnetresonanztomographie werden Hochfrequenzimpulse verwendet, um Magnetresonanzsignale als Messsignale zu erzeugen. In der Spektroskopie werden üblicherweise FIDs ausgelesen und in der Bildgebung Echosignale.

Dabei wird der sogenannte k-Raum abgetastet. Je nach Art der Abtastung spricht man von einer kartesischen, radialen oder spiralen Abtastung. Im Folgenden wird o.B.d.A. eine kartesische Abtastung beschrieben. Bei dieser werden k-Raum-Zeilen aufgenommen, die im Bildraum in Leserichtung $G_R$ verlaufen. Diese Richtung wird im k-Raum mit $k_x$ bezeichnet.

Eine Messsequenz weist drei Phasen auf: die Anregungsphase, die Evolutionsphase und die Detektionsphase.

In der Anregungsphase kann ein Präparationsmodul vorhanden sein, um die Magnetisierung in einer gewünschten Wiese zu wichten. In der Detektionsphase werden eine oder mehrere k-Raum-Zeilen ausgelesen.

Wird in der Detektionsphase eine einzige k-Raum-Zeile ausgelesen, so entspricht dies einer üblichen Aufnahmestrategie, die z.B. bei einem Spinecho, einem Gradientenecho oder der schnelleren Version in Form eines FLASH eingesetzt wird.

Wird mehr als eine k-Raum-Zeile in einem Teilexperiment aber nicht alle aufgenommen spricht man von einer segmentierten Messung. Dies kann vorkommen, da physikalische oder medizinische Gründe die Aufnahme nur eines Teils der Messdaten erlauben. Physikalische Gründe sind ein kurzes $T_2$ oder $T_2^*$. Dementsprechend können ein Fast Spin Echo mit dem Akronym FSE oder ein EPI, kurz für echoplanar imaging, als Messsequenzen mit mehreren k-Raum Segmenten ausgestaltet sein.

Medizinische Gründe sind bspw. ein zu kurzer Herzpuls, d.h. dass die Messzeit durch den Herzschlag begrenzt ist.

Kann man alle benötigten k-Raum-Zeilen in einem Zug aufnehmen spricht man auch von einer single-shot-Sequenz. Dies ist mit EPI und FSE bei ausreichend langen Relaxationszeiten ebenfalls möglich.

Die aufzunehmende Menge an Messdaten ist dabei durch das sogenannte Nyquist-Theorem gegeben:

$$\Delta k \; < \; 2\pi/L.$$

Dabei ist $\Delta k$ der Abstand der Signale im k-Raum und L die Ausdehnung des Objektes in der darzustellenden Dimension.

Um eine Verringerung der Messdauer zu erzielen wurden zuerst Messsequenzen mit verringerter Messzeit wie FLASH, FSE oder TrueFISP entwickelt. Diese erfüllen bei der Messdatenaufnahme das Nyquist-Theorem weiterhin.

Um eine weitere Beschleunigung zu erzielen wurde es notwendig, die Schranken des Nyquist-Theorems aufzuheben. Dies ist möglich, da das Nyquist-Theorem lediglich eine Bandbegrenzung des abzutastenden Signals voraussetzt. Es wird aber keinerlei sonstiges Vorwissen vorausgesetzt oder verwendet.

Bei der parallelen Bildgebung wird nur ein Teil der zur Erfüllung des Nyquist-Theorems aufzunehmenden k-Raum-Daten aufgenommen, dafür aber mit mehreren Spulen. Der k-Raum ist also unterabgetastet. Dies führt zu Einfaltungen, auch Aliasing-Artefakt genannt.

Um diese zu vermeiden ist bei der Rekonstruktion ein spezielles Rekonstruktionsverfahren verwenden. Das SENSE-Verfahren entfaltet die Messdaten im Bildraum. Beim sogenannten GRAPPA-Verfahren werden die fehlenden k-Raum-Zeilen dagegen mit Hilfe von Kalibrierungsdaten rekonstruiert. Die Kalibrierungsdaten sind ein vollständiger Messdatensatz im Hinblick auf das Nyquist-Theorem. Die Kalibrierungsdaten können einen Teil oder den ganzen k-Raum umfassen.

Eine Weiterentwicklung des Abtastschemas für die parallele Bildgebung bei mehreren Schichten ist das sogenannte CAIPIRINHA, ein Akronym für Controlled Aliasing In Parallel Imaging Results IN Higher Acceleration. Dabei werden die Einfaltungsartefakte gezielt modifiziert, um die Bildrekonstruktion zu verbessern.

Beim zuerst veröffentlichten MS-CAIPRINHA (Breuer F.A., Blaimer M., Heidemann R.M., Müller M.F., Griswold M.A. and Jakob P.M.: Controlled Aliasing In Parallel Imaging Results IN Higher Acceleration for Multi-Slice Imaging, Magn. Res. Med. 53: 684-691) wird eine Modifikation bei zwei Schichten dadurch erreicht, dass zwei Schichten mit alternierenden Dualband-Impulsen angeregt werden.

Eine Weiterentwicklung stellt das sogenannte blipped CAIPIRINHA (Setsompop K., Gagoski B.A., Polimeni J.R., Witzel T., Van Wedeen J., and Wald L.L.: Blipped-Controlled Aliasing in Parallel Imaging (blipped-CAIPI) for simultaneous multi-slice EPI with reduced g-factor penalty, Magn. Res. Med. 67: 1210-1224) dar, bei dem während bzw. vor dem Auslesen der Messsignale der Schichtgradient mittels Gradientenblips oszillierend geschaltet wird.

Auch bei dieser Art der Abtastung werden Kalibrierungsdaten zur Auswertung benötigt.

Es ist daher Aufgabe der vorliegenden Erfindung, ein Verfahren zur Aufnahme eines Magnetresonanzdatensatzes anzugeben, bei dem der k-Raum mit einem blipped CAIPIRINHA-Verfahren abgetastet wird und die Messdatenaufnahme effizienter ausgestaltet ist.

Diese Aufgabe wird gelöst durch ein Verfahren zur Aufnahme eines Magnetresonanzdatensatzes der eingangs genannten Art, wobei die Messsignale in einem ersten Bereich des k-Raums mit einer ersten Abtastdichte und in einem zweiten Bereich des k-Raums mit einer zweiten Abtastdichte aufgenommen werden.

Als Kern der Erfindung wird dabei angesehen, eine segmentierte Messsequenz so auszugestalten, dass mehr Daten als die zur Erstellung eines Bilddatensatzes notwendigen Daten aufgenommen werden und so eine Zusatzmessung, nämlich die Kalibrierungsmessung, einzusparen.

Eine segmentierte Aufnahme kann man auch als Aufnahme mit mehreren Segmenten oder auch mit mehreren Teilexperimenten bezeichnen. Ein Segment bezeichnet hierbei eine Teilaufnahme, welche vorteilhafterweise als Echozug ausgestaltet ist. Die Anordnung der Segmente im k-Raum kann einen zusammenhängenden Bereich umfassen. Alternativ kann bspw. bei einer zehnsegmentigen Aufnahme das erste Segment alle zehnten k-Raum-Positionen startend mit Position eins, das zweite Segmente alle zehnten k-Raum-Positionen startend mit Position zwei usw. umfassen. Dies kann man als versetze Abtastung bezeichnen.

Als Maß für die Abtastdichte kann man $\Delta k$, also den Abstand der Signale im k-Raum, verwenden.

Bei der Aufnahme von Echosignalen kann die Abtastdichte in $k_y$- und $k_z$-Richtung variiert werden, bei der Aufnahme von FIDs zusätzlich in $k_x$-Richtung.

Als Bereiche des k-Raums werden dabei die aufeinanderfolgenden k-Raum-Punkte angesehen, die mit der gleichen Abtastdichte abgetastet wurden. Dabei muss mindestens ein Wechsel der Abtastdichte stattfinden, grundsätzlich können es aber sehr viel mehr sein.

Begrenzt wird die theoretische Anzahl an Bereichen durch die Anzahl der aufzunehmenden k-Raum-Signale und die Segmentierung.
Eine simultaneous multislice (SMS) Aufnahme mit CAIPIRINHA blipping kann als 3D Aufnahme gesehen werden, so wie in Setsompop et al. beschrieben wurde. Ein Wechsel in $k_z$-Richtung wird mittels Gradientenblip, also eines Gradienten in Schichtauswahlrichtung, vollzogen. Dabei muss $k_z$ nicht bei jedem folgenden Messsignal wechseln, es muss aber wenigstens einen Wechsel pro Segment geben. Je höher die Anzahl der Wechsel, desto besser ist das SNR.
Der Abstand der k-Raum-Signale in $k_x$-Richtung ist vorteilhafterweise immer gleich. In $k_y$-Richtung hängt die Abtastdichte vom Beschleunigungsfaktor und vom field of view (FOV) ab. In $k_z$-Richtung hängt die Abtastdichte vom SMS-Faktor und vom FOV-shift-Faktor ab. Der SMS-Faktor ist die Anzahl der gleichzeitig angeregten Schichten. Der FOV-shift-Faktor bezeichnet den Versatz der gleichzeitig angeregten Schichten voneinander, und zwar normiert auf das FOV. Der Wechsel wird wie beschrieben durch die Blipgradienten erreicht.

Demgegenüber ist in der vorliegenden Erfindung vorgesehen, die Abtastdichte in $k_y$- und/oder $k_z$-Richtung zu variieren.

Vorteilhafterweise kann die Abtastdichte in $k_y$-Richtung verändert werden. Dies erfolgt durch die entsprechende Einstellung des Phasenkodiergradienten.

Alternativ oder zusätzlich kann die Abtastdichte in $k_z$-Richtung verändert werden. Bei einem blipped CAIPIRINHA-Verfahren liegen in z-Richtung Blipgradienten an, um zwischen den k-Raum-Zeilen in $k_z$-Richtung zu wechseln. Durch ein zusätzliches Schalten oder Weglassen der Blipgradienten kann die Abtastdichte in z-Richtung aber auch in Kombination mit dem Phasenkodiergradienten in $k_y$-Richtung eingestellt werden.

Alternativ oder zusätzlich kann die Abtastdichte in $k_x$-Richtung verändert werden. Diese Möglichkeit ergibt sich bei der Aufnahme von FIDs.

Der Magnetresonanzdatensatz wird mit mehreren Spulen aufgenommen. Mit anderen Worten wird parallele Bildgebung bzw. eine parallele Aufnahme verwendet. Bevorzugt wird zur Rekonstruktion eines Bilddatensatzes aus dem Magnetresonanzdatensatz ein GRAPPA-Verfahren verwendet. Einfaltungen werden also durch eine Rekonstruktion fehlender Daten im k-Raum vermieden.

Der Magnetresonanzdatensatz kann auch als Rohdatensatz bezeichnet werden. Er ist durch mehrere Rekonstruktionsschritte wie bspw. eine Fouriertransformation oder auch das bereits erwähnte GRAPPA-Verfahren zur Vermeidung von Einfaltungen in einen Bilddatensatz zu überführen.

Bevorzugt können die Messsignale in einem dritten Bereich mit einer dritten oder der ersten Abtastdichte aufgenommen werden. Es kann grundsätzlich wie beschrieben eine größere Anzahl an Bereichen mit gleicher Abtastdichte geben. Bevorzugt ist aber, dass eine ungerade Anzahl an Bereichen vorliegt. Vorzugsweise können wenigstens, insbesondere genau, drei Bereiche vorhanden sein.

Vorzugsweise können die Bereiche axialsymmetrisch zur Mitte des k-Raums bezüglich der $k_y$-Richtung angeordnet sein. Eine symmetrische Anordnung bedeutet, dass die ersten bspw. zehn und die letzten zehn k-Raum-Zeilen mit einer ersten Abtastdichte und die k-Raum-Zeilen dazwischen mit einer zweiten Abtastdichte aufgenommen werden. Die dabei vorkommenden Wechsel in $k_z$-Richtung bleiben unberücksichtigt.

Bevorzugt können die Bereiche symmetrisch zur Mitte des k-Raums bezüglich der $k_z$-Richtung angeordnet sein.

Vorteilhafterweise kann der zweite Bereich die Mitte des k-Raums umfassen. Insbesondere kann der zweite Bereich die Mitte des k-Raums in $k_y$-Richtung und/oder $k_z$-Richtung umfassen.

Im Folgenden wird bei der Beschreibung der Abtastung das Vorgehen in $k_y$-Richtung beschrieben außer es ist explizit anders erwähnt. In $k_z$-Richtung findet üblicherweise ein Wechsel des $k_z$-Werts statt und in $k_x$-Richtung wird ein Echosignal abgetastet.

Die Abtastung des k-Raums kann bei segmentierten Messungen mit unterschiedlichen Strategien erfolgen. Man kann entweder ausgehend vom Rand des k-Raums anfangen, ausgehend vom Zentrum des k-Raums oder ab dem zweiten Segment bzw. Echozug dort, wo die k-Raum-Abtastung des ersten oder allgemeiner letzten Segmentes bzw. Echozug endete. Damit ist also festgelegt, dass ein Wechsel der Abtastdichte nicht genau in der Mitte des k-Raums erfolgt.

Bevorzugt kann die zweite Abtastdichte höher sein als die erste Abtastdichte. Durch die höhere Abtastdichte kann in einem oder mehreren Segmenten oder Echozügen eine vollständige Abtastung in diesem Bereich erzielt werden. Vollständig bezieht sich dabei wieder auf das Nyquist-Theorem. Insbesondere kann die zweite Abtastdichte doppelt so hoch oder viermal so hoch sein wie die erste und/oder dritte Abtastdichte.

Vorteilhafterweise können in jedem Segment die Messsignale oder die zusätzlichen Messsignale des zweiten Bereichs vor den Messsignalen des ersten Bereichs und/oder vor den Messsignalen des dritten Bereichs aufgenommen werden. Am Anfang der Messung ist das Signal am höchsten, dadurch haben die Kalibrierungssignale das größte SNR.

Alternativ können in jedem Segment die Messsignale des zweiten Bereichs nach den Messsignalen des ersten Bereichs und vor den Messsignalen des dritten Bereichs aufgenommen werden. Dann werden die zusätzlichen Messsignale im zweiten Bereich zwischendurch eingeschoben.

Weiter alternativ können in jedem Segment die Messsignale oder die zusätzlichen Messsignale des zweiten Bereichs nach den Messsignalen des ersten Bereichs und nach den Messsignalen des dritten Bereichs aufgenommen werden. Dadurch bleibt die eigentliche Messung unverändert und das Restsignal am Ende der Messung wird noch für Kalibrierungszwecke genutzt.

Vorteilhafterweise können die Messsignale des zweiten Bereichs nach Durchlauf mindestens zweier Segmente einen vollständigen Satz an Kalibrierungsdaten ergeben. Dann stehen alle mindestens zwei Segmente Kalibrierungsdaten zur Verfügung. Dies ist insbesondere bei bewegten Untersuchungsbereichen vorteilhaft.

Vorteilhafterweise kann als Messsequenz ein Fast Spin Echo (FSE) verwendet werden. Dabei entsteht die Segmentierung automatisch durch die Echozüge. Alternativ kann als Messsequenz ein EPI verwendet werden. Weiter alternativ kann als Messsequenz ein FLASH verwendet werden. Weiter alternativ kann als Messsequenz ein TrueFISP verwendet werden.

Vorzugsweise können die Messsignale als Echosignale ausgebildet sein. Mit anderen Worten wird eine bildgebende Sequenz verwendet bzw. während des Auslesens der Messsignale liegt ein Lesegradient an.

Bevorzugt kann der k-Raum im zweiten Bereich spätestens nach Durchlauf aller Segmente vollständig abgetastet sein. Ziel ist es wie beschrieben, die k-Raum-Zeilen im zweiten Bereich zur Kalibrierung zu verwenden. Dann sind sie vollständig im Sinne einer Ermöglichung einer Kalibrierung aufzunehmen.

Vorteilhafterweise kann der k-Raum im zweiten Bereich wenigstens zweimal vollständig abgetastet werden. Weiterhin kann der k-Raum im zweiten Bereich nach der Messung jedes zweiten Segmentes vollständig abgetastet sein. Insbesondere kann der k-Raum im zweiten Bereich nach der Messung jedes Segmentes abgetastet sein. Je öfter eine vollständige Abtastung des k-Raums im zweiten Bereich erfolgt, desto besser kann bei Bewegungen die Kalibrierung erfolgen. Ist nach der Messung zweier Segmente der zweite Bereich vollständig abgetastet wird nicht aufgehört, k-Raum-Zeilen im zweiten Bereich aufzunehmen.

Während der Anregungsphase können Präparationsmodule eingesetzt werden. Die Anregungsphase schließt mit dem letzten HF-Impuls zur Anregung ab. Bei einem Spinecho besteht die Anregungsphase lediglich aus dem 90°-Impuls, bei einem FLASH aus dem HF-Impuls.

Bevorzugt weist die Anregungsphase das Anlegen eines Anregungsimpulses, insbesondere eines einzigen Anregungsimpulses, auf. Dieser kann eine 2D-Schicht oder mehrere Schichten anregen.

Der Anregungsimpuls kann vorteilhafterweise als Mehrbandimpuls, insbesondere als Dualbandimpuls, ausgestaltet sein. Dann kann der Anregungsimpuls zwei bzw. mehrere Schichten gleichzeitig anregen. Vorzugsweise kann während des Anliegens des Anregungsimpulses gleichzeitig ein Schichtauswahlgradient angelegt sein. Dies ist unabhängig von der Pulsform möglich.

Der Anregungsimpuls ist vorzugsweise phasenmoduliert.

Die Schichten, die gleichzeitig akquiriert werden, liegen vorteilhafterweise parallel, d.h. sie kreuzen sich nicht.

In der Evolutionsphase können sich bspw. wenigstens ein Schicht-Rephasiergradient und/oder wenigstens ein Phasenkodiergradient und/oder wenigstens ein Lesedephasiergradient und/oder wenigstens ein Refokussierungsimpuls, etc. finden. In der Detektionsphase ist bei bildgebenden Sequenzen üblicherweise wenigstens ein Lesegradient angelegt, außerdem ist das Akquisitionsfenster offen.

In einer Sequenz können auf eine Anregungsphase auch mehrere Evolutions- und Detektionsphasen folgen, wie es z.B. beim FSE der Fall ist. Ansonsten werden die Phasen solange wiederholt, bis alle Messsignale aufgenommen sind.

Da die Erfindung in unterschiedlichen Messsequenzen verwendet werden kann, werden folgende Definitionen vorgenommen:

Als Messsequenz wird wie üblich eine Abfolge von HF-Impulsen, Gradientenfeldern, Wartezeiten und Akquisitionsfenstern bezeichnet, die den Ablauf der Messsequenz genau festlegen und charakterisieren. Beispiele für Messsequenzen sind die bereits angesprochenen FLASH, Spinecho, Gradientenecho, FSE, EPI, TrueFisp, etc. Diese daraus ermittelten Bilddatensätze können durch Präparationsmodule Wichtungen aufweisen.

Eine Messsequenz besteht dabei aus definierten oder definierbaren Teilexperimenten, den Segmenten. Bei einem Fast Spin Echo werden nach einem Anregungsimpuls mehrere Refokussierungsimpulse verwendet, weswegen von einem Echozug gesprochen wird.

Diese Ausführungen sollen insbesondere die bestehenden fachmännischen Konventionen darlegen.

Da bei der Datenaufnahme mehrere Spulen zum Einsatz kommen, wird von jeder Spule nur ein Teil eines vollständigen Satzes an k-Raum-Daten aufgenommen. Die fehlenden Daten können mit dem weiter oben beschriebenen GRAPPA-Verfahren berechnet werden.

Vorzugsweise kann die Evolutionsphase wenigstens einen, insbesondere genau einen, Refokussierungsimpuls aufweisen. Durch das mehrmalige Vorkommen von Evolutions- und Detektionsphase erzeugt man ein FSE.

Zum Einstrahlen des Anregungsimpulses und/oder der Refokussierungsimpulse können eine Bodycoil oder ein Spulenarray verwendet werden. Das Auslesen der Echosignale erfolgt mittels eines Spulenarrays.

Vorzugsweise kann gleichzeitig zum Anregungsimpuls und/oder Refokussierungsimpuls bzw. den Refokussierungsimpulsen jeweils ein Schichtauswahlgradient anliegen. Dieser ermöglicht eine schichtselektive Anregung.

Vorzugsweise kann nach dem Auslesen eines Echosignals in einer Detektionsphase ein Phase-Rewind-Gradient angelegt werden. Dieser soll die Magnetisierung wieder in die Mitte des k-Raums bringen.

Die Lösung der eingangs genannten Aufgabe wird außerdem durch ein Computerprogrammprodukt bzw. ein Computerprogramm erzielt, das zur Steuerung einer Steuerungseinrichtung eingesetzt werden kann, welche eine Bilderzeugungseinheit einer Magnetresonanzanlage steuert, welches das vorgenannte erfindungsgemäße Verfahren ausführt.

Daneben betrifft die Erfindung einen Datenträger für eine Steuerungseinrichtung zur Steuerung einer Datenerzeugungseinheit einer Magnetresonanzanlage mit Daten zum Durchführen des beschriebenen Verfahrens. Vorteilhafterweise kann die Datenerzeugungseinheit eine Bilderzeugungseinheit sein.

Daneben betrifft die Erfindung eine Magnetresonanzanlage mit einer Steuerungseinrichtung. Die Magnetresonanzanlage zeichnet sich dadurch aus, dass die Steuerungseinrichtung zur Durchführung des Verfahrens wie beschrieben ausgebildet ist.

Die Implementierung der vorgenannten Verfahren in der Steuervorrichtung kann dabei als Software oder aber auch als (fest verdrahtete) Hardware erfolgen.

Weitere vorteilhafte Ausgestaltungen der erfindungsgemäßen Magnetresonanzanlage korrespondieren zu entsprechenden Ausgestaltungen des erfindungsgemäßen Verfahrens. Zur Vermeidung unnötiger Wiederholungen wird somit auf die entsprechenden Verfahrensmerkmale und deren Vorteile verwiesen.

Weitere Vorteile, Merkmale und Besonderheiten der vorliegenden Erfindung ergeben sich aus der nachfolgenden Beschreibung vorteilhafter Ausgestaltungen der Erfindung.

Dabei zeigen:

Fig. 1　　eine Magnetresonanzanlage,

Fig. 2　　eine segmentierte Messsequenz,

Fig. 3　　ein erstes k-Raum-Abtastschema,

Fig. 4　　ein zweites k-Raum-Abtastschema,

Fig. 5　　ein drittes k-Raum-Abtastschema, und

Fig. 6　　ein Ablaufschema zur Aufnahme eines Magnetresonanz-datensatzes.

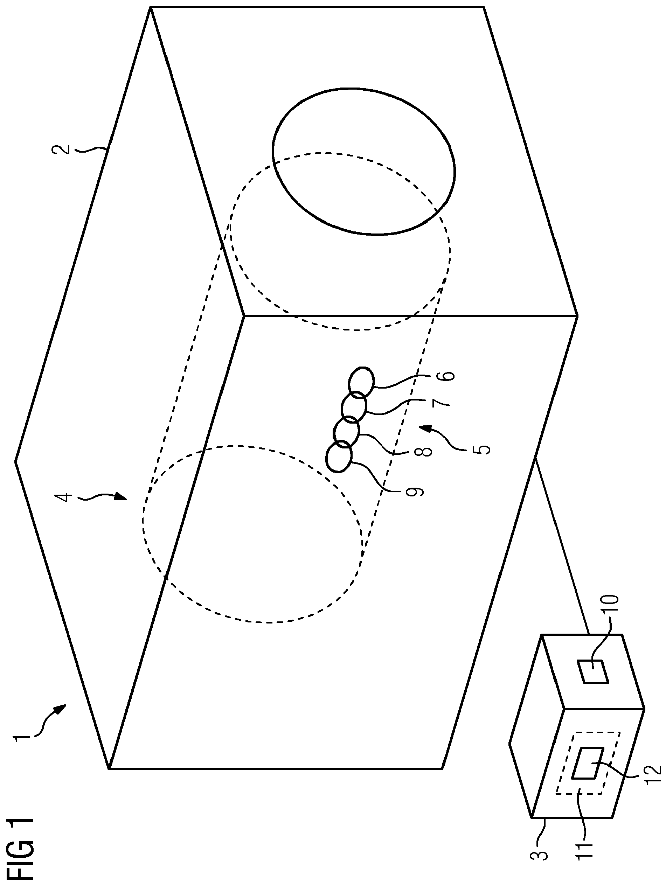

Figur 1 zeigt eine Magnetresonanzanlage 1. Diese umfasst im Wesentlichen ein Magnetresonanzgerät 2 und eine Steuerungseinrichtung 3.

Im Magnetresonanzgerät 2 ist eine Sendespulenanordnung 4 angeordnet. Die Sendespulenanordnung 4 kann als sogenannte Bodycoil ausgebildet sein. Alternativ kann die Sendespulenanordnung als Spulenarray ausgebildet sein.

Als Empfangsspulenanordnung 5 ist ein Spulenarray mit Spulen 6, 7, 8 und 9 vorhanden. Das Spulenarray kann selbstverständlich auch eine andere Anzahl an Spulen aufweisen. Mit dem Spulenarray 5 können Messsignale mittels paralleler Bildgebung aufgenommen werden.

In einer weiteren möglichen Ausgestaltung können die Sendespulenanordnung 4 und die Empfangsspulenanordnung 5 durch die gleichen Spulen gebildet werden.

Das Spulenarray 5 wird bei der dargestellten Ausgestaltung aber nur zum Auslesen des Messsignals verwendet.

Die Steuerungsrichtung 3 umfasst einen Prozessor 10, einen Datenträger 11 und wenigstens ein darauf gespeichertes Computerprogrammprodukt 12. Messsequenzen können als Computerprogrammprodukte 12 ausgebildet sein.

Weitere Bestandteile der Magnetresonanzanlage 1 wie bspw. Gradientenspulen oder eine Patientenliege sind der Übersichtlichkeit halber nicht dargestellt.

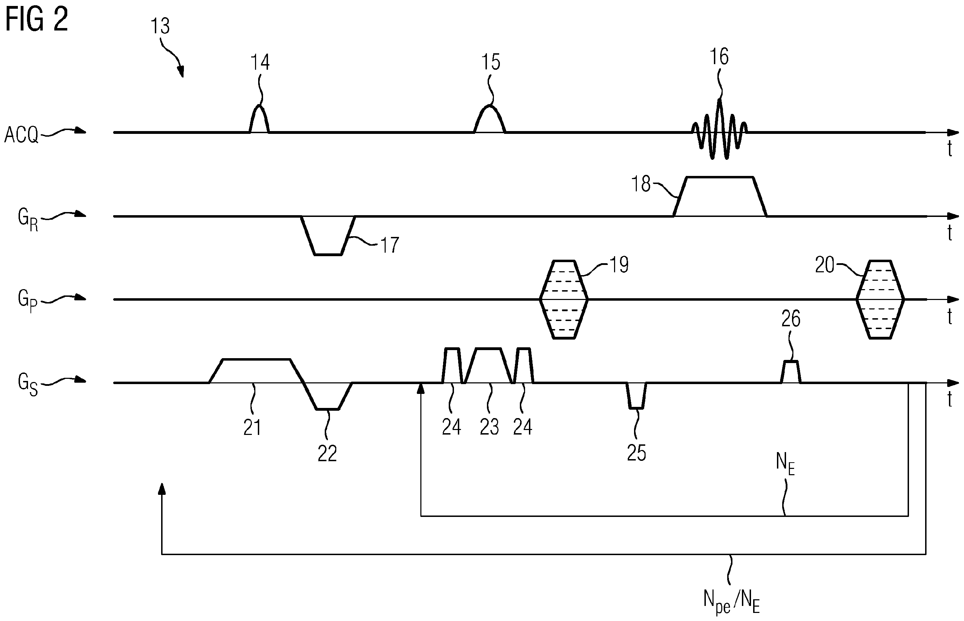

Figur 2 zeigt ein Sequenzdiagramm 13 einer FSE-Messsequenz zur Aufnahme eines Magnetresonanzdatensatzes. Mit der FSE-Messsequenz werden zwei Schichten gleichzeitig gemessen, bei der Abtastung des k-Raums wird ein blipped CAIPIRINHA-Verfahren eingesetzt.

Entlang der Achse ACQ sind die Hochfrequenzimpulse und die Akquisitionsfenster eingezeichnet.

Der Anregungsimpuls 14 ist als Dualbandimpuls ausgestaltet. Er hat einen Flipwinkel von 90°. Der Refokussierungsimpuls 15 besitzt dagegen einen Flipwinkel von 180°. Der Anregungsimpuls 14 und der Refokussierungsimpuls 15 erzeugen das Echosignal 16.

Durch das $N_E$-fache anlegen des Refokussierungsimpulses 15 wird ein Echozug aus $N_E$ Echosignalen 16 erzeugt.

In Leserichtung $G_R$ liegen der Lese-Dephasiergradient 17 und der Lesegradient 18 an.

In Phasenrichtung $G_P$ liegen ein Phasenkodiergradient 19 und ein Phase-Rewind-Gradient 20 an. Der Phase-Rewind-Gradient 20 kompensiert das Gradientenmoment den Phasenkodiergradienten 19, sodass die Gesamtphase in Phasenrichtung zwischen zwei Refokussierungsimpulsen 15 gleich Null ist.

Da $N_E$ Echosignale pro Echozug aufgenommen werden nur $N_{pe}/N_E$ Teilexperimente bzw. Segmente bzw. Echozüge durchlaufen. $N_{pe}$ ist dabei die Anzahl der phasenkodierenden Schritte.

In Schichtrichtung $G_s$ liegt ein Schichtauswahlgradient 21 gleichzeitig zum Anregungsimpuls 14 an. Auf diesen folgt ein Schicht-Rephasiergradient 22.

Parallel zum Refokussierungsimpuls 15 liegt ein Schichtauswahlgradient 23 an. Dieser ist von Crushergradienten 24 umgeben. Die Crushergradienten 24 sollen die Detektion von ungewollten Echosignale aus dem Refokussierungsimpuls 15 vermeiden.

Die Gradientenblips 25 und 26 sind nach jedem zweiten Refokussierungsimpuls angelegt und bewegen den Ausleseort im k-Raum in $k_z$-Richtung. Der Gradientenblip 25 ermöglicht also einen Wechsel im k-Raum in die $k_z$-Richtung und der Gradientenblip 26 sorgt für eine Rückkehr zur ursprünglichen k-Raum Position. Bei mehr als zwei Schichten sind entsprechend die Gradientenblips 25 und 26 vom Gradientenmoment her anzupassen.

Je nach FOV-Shift- und R-Faktor sowie Trajektorie des k-Raum-Durchlaufs muss das nicht unbedingt bei jedem zweiten Hochfrequenzimpuls der Fall sein. Die Gradientenblips können also auch nach jedem dritten oder vierten oder fünften ... Refokussierungsimpuls angelegt sein.

Der Gradientenblip 25 kann auch früher angelegt werden und bspw. mit einem der Crushergradienten 24 verschmolzen werden.

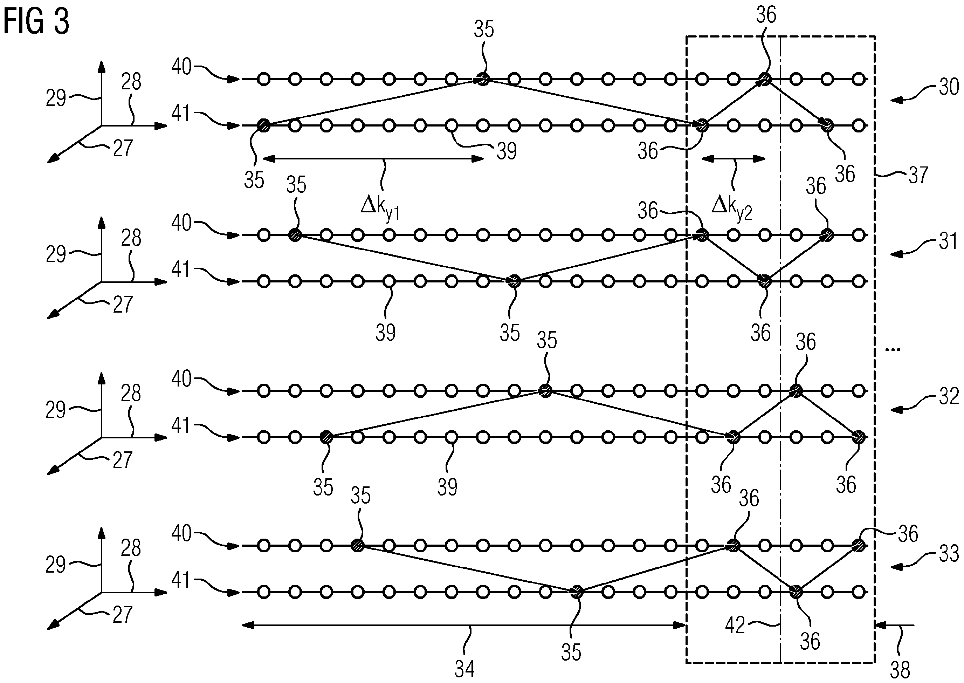

Figur 3 zeigt ein Abtastschema für eine segmentierte Messsequenz. Dabei zeigen jeweils die Achsen 27 die $k_x$-Richtung, die Achsen 28 die $k_y$-Richtung und die Achsen 29 die $k_z$-Richtung. Die k-Raum-Zeilen liegen in $k_x$-Richtung und sind daher als Punkt abgebildet. Die akquirierten k-Raum-Zeilen sind schwarz gefüllt und die ausgelassenen k-Raum-Zeilen weiß gefüllt dargestellt.

Rein exemplarisch sind die Segmente 30, 31, 32 und 33 gezeigt. Mit der FSE-Messsequenz können aber natürlich beliebig viele Segmente akquiriert werden.

Im ersten Bereich 34 ist die Abtastdichte $\Delta k_{y1}$ der akquirierten Messsignale 35 offensichtlich niedriger als die Abtastdichte $\Delta k_{y2}$ der akquirierten Messsignale 36 im zweiten Bereich 37. An den zweiten Bereich 37 schließt ein dritter Bereich 38 an.

Es sind im ersten Bereich 34 und im zweiten Bereich 37 lediglich exemplarisch Messsignale dargestellt, die insbesondere die Abtastdichte veranschaulichen sollen. Insbesondere die Anzahl der "übersprungenen" k-Raum-Zeilen 39 und die Anzahl der akquirierten Messsignale 35 und 36 sind nicht absolut anzusehen.

Relevant sind aber der Wechsel im k-Raum in die $k_z$-Richtung zwischen den Zeilen 40 und 41 zwischen der Aufnahme zweier Messsignale 35 und/oder 36. Dieser wird durch die Gradientenblips 25 und 26 erreicht. Das Fortschreiten in $k_y$-Richtung bewirkt dagegen der Phasenkodiergradient 19.

Werden alle Segmente 30 bis 33 und ggf. die darauffolgenden Segmente durchlaufen so erhält man im ersten Bereich 34, der am Rand des k-Raums liegt und sich in Richtung k-Raum-Mitte erstreckt, insgesamt ein Zick-Zack-Muster abgetastet. Das heißt, dass in jeder der Zeilen 40 und 41 jede zweite $k_y$-Raum-Zeile abgetastet ist. Die abgetasteten k-Raum-Zeilen in Form der Messsignale 35 sind um $\Delta k_y$ gegeneinander versetzt. In ersten Bereich erhält man so eine blipped CAIPIRINHA-Abtastung.

Im zweiten Bereich erhält man dagegen nach vier Segmenten 30, 31, 32 und 33 einen vollständig abgetasteten zweiten Bereich 37. Dieser kann zur Kalibrierung bei einer GRAPPA-Rekonstruktion verwendet werden.

Der zweite Bereich 37 liegt symmetrisch, genauer gesagt achsensymmetrisch, um die Mitte 42 des k-Raums in $k_y$-Richtung. In $k_z$-Richtung liegt keine Achsensymmetrie vor. Die Mitte 42 gilt als vom zweiten Bereich 37 eingeschlossen, wenn zumindest die angrenzenden k-Raum-Zeilen mit einer höheren Abtastdichte $\Delta k_{y2}$ abgetastet sind.

Im dritten Bereich 38 wird die gleiche Abtastdichte $\Delta k_{y1}$ wie im ersten Bereich 34 verwendet. Der dritte Bereich 38 ist aus Platzgründen jedoch nur angedeutet.

Bei der in Figur 3 gezeigten Abtastung werden die im zweiten Bereich 37 aufgenommenen Messsignale 36 in der Mitte der Messung jeweils eines Segmentes akquiriert. Zuerst werden also die äußeren k-Raum-Zeilen im ersten Bereich 34 aufgenommen, dann zur Mitte 42 des k-Raums hin weitergegangen, dort die k-Raum-Zeilen 36 im zweiten Bereich 37 akquiriert und dann wieder nach außen hin gewandert, um die k-Raum-Zeilen des dritten Bereichs 37 aufzunehmen.

Die Abtastdichte $\Delta k_{y2}$ im zweiten Bereich 37 kann aber auch höher oder niedriger als gezeigt gewählt werden. Sie kann so gewählt werden, dass bereits nach jeweils zwei Segmenten 30 und 31 oder 32 und 33 ein vollständig abgetasteter zweiter Bereich 37 vorliegt. Alternativ kann sie so gewählt werden, dass die vollständige Abtastung des zweiten Bereichs 37 nach Aufnahme aller Segmente 30 bis 33 erreicht ist.

Zusammenfassend sei nochmals darauf hingewiesen, dass nach dem Durchlauf aller Segmente 30, 31, 32, und 33 der erste Bereich 34 und der dritte Bereich 38 unterabgetastet sind, der zweite Bereich 37 aber nicht.

Nur der Vollständigkeit halber wird ausgeführt, dass zuerst alle Messsignale 35 und 36 des ersten Segments 30 aufgenommen werden, dann alle Messsignale 35 und 36 des zweiten Segments 31, daraufhin alle Messsignale 35 und 36 des dritten Segments 32 und so weiter bis zum letzten Segment, hier das Segment 33.

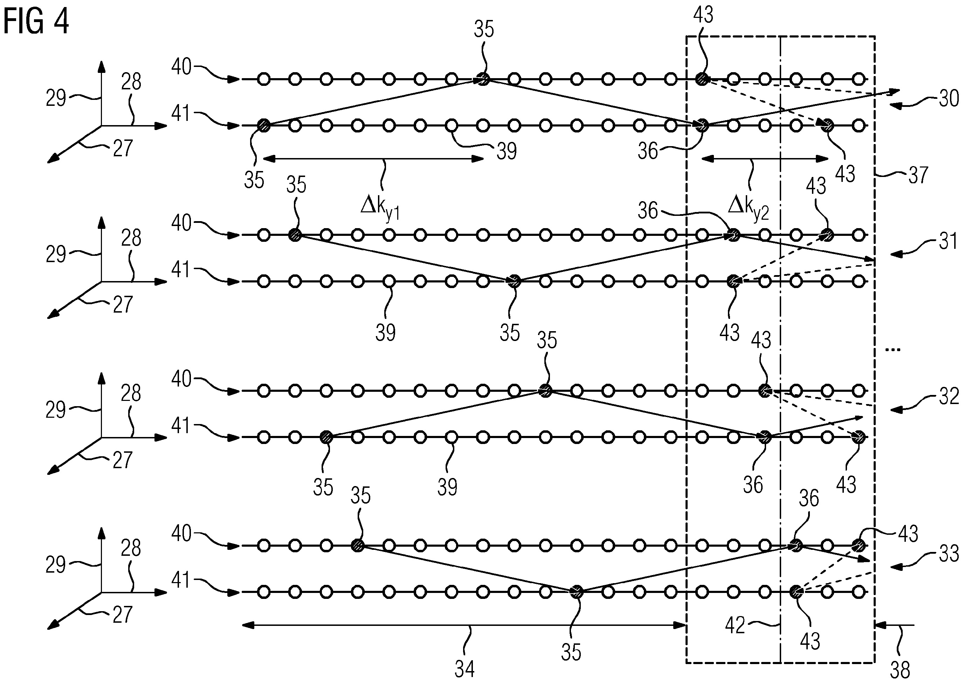

Figur 4 zeigt eine alternative Vorgehensweise zur Aufnahme von Messsignalen 43 zur Kalibrierung. Dabei werden die Messsignale 35 des ersten Bereichs 34, die Messsignale 36 des zweiten Bereichs 37 du die Messsignale des dritten Bereichs 38 mit einer ersten Abtastdichte $\Delta k_{y1}$ abgetastet. Es wird also das erste Segment 30 anfangs so abgetastet wie bei einer bekannten blipped CAIPIRINHA-Abtastung. Im Anschluss daran werden im zweiten Bereich 37 noch ein oder mehrere zusätzliche Messsignale 43 zu Kalibrierungszwecken aufgenommen. Wieviele zusätzliche Messsignale 43 aufgenommen werden hängt einerseits davon ab, wieviel SNR das Messsignals im Hinblick auf $T_2$-Zerfall noch hat. Andererseits ist natürlich zu berücksichtigen, ob die Kalibrierungsdaten bereits nach der Aufnahme eines oder zweier Segmente vollständig sein sollen oder erst nach der Aufnahme aller Segmente.

In einer weiter alternativen Vorgehensweise können die zusätzlichen Messsignale 43 auch am Anfang der Messung eines Segmentes aufgenommen werden.

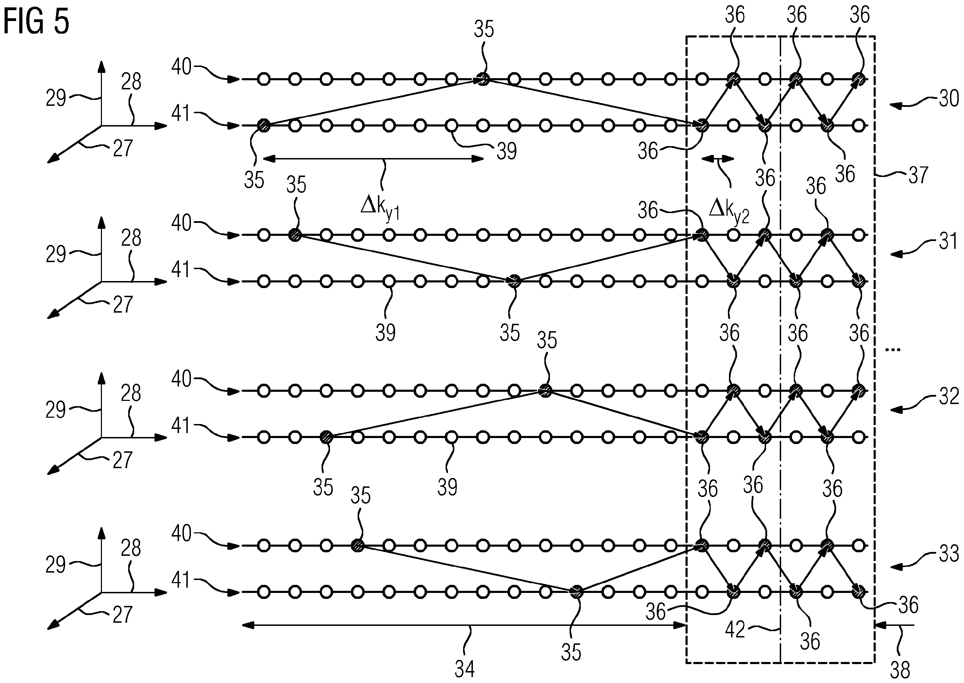

Figur 5 zeigt zur Verdeutlichung der Änderung der Abtastdichte $\Delta k_{y2}$ ein Vorgehen, bei dem bereits nach der Messung zweier Segmente 30 und 31 oder 32 und 33 vollständige Kalibrierungsdaten vorliegen. Dabei ist die Abtastdichte doppelt so hoch wie in Figur 3, ansonsten stimmen die Figuren überein.

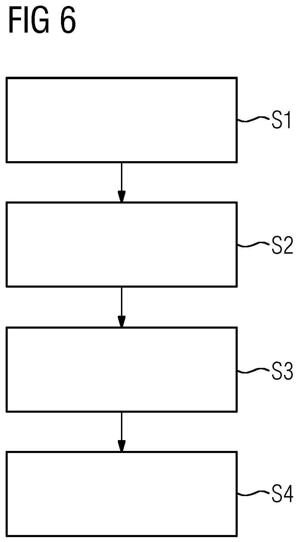

Zusammenfassend kann also ausgehend von einer blipped CAIPIRINHA-Abtastung bei segmentieren Messungen folgendermaßen vorgegangen werden, um während der Aufnahme eines Bilddatensatzes mit zwei oder mehr Schichten gleichzeitig Kalibrierungsdaten zu erhalten, wie Figur 6 zeigt:

Zuerst wird in Schritt S1 ein zweiter Bereich 37 festgelegt, der vollständig, d.h. nicht unterabgetastet, abgetastet werden soll. Hierzu gehören die Richtung und die Anzahl der k-Raum-Zeilen. Im den Figuren 3 bis 5 sind es sechs k-Raum-Zeilen in $k_y$-Richtung, und zwar die mittleren k-Raum-Zeilen. Bevorzugt sind wenigstens 16 k-Raum-Zeilen, insbesondere in $k_y$-Richtung.

Daraus ergibt sich automatisch, dass die äußeren vierzehn k-Raum-Zeilen im ersten Bereich 34 und die äußeren vierzehn k-Raum-Zeilen im dritten Bereich 38 unterabgetastet aufgenommen werden wie bei blipped CAIPIRINHA vorgesehen. Die Anzahl der k-Raum-Zeilen im ersten Bereich 34 und dritten Bereich 38 ergibt sich automatisch aus der Gesamtzahl der k-Raum-Zeilen und der Anzahl der k-Raum-Zeilen im zweiten Bereich 37.

Im zweiten Schritt S2 wird die Abtastdichte $\Delta k_{y2}$ ausgesucht. Diese wird so gewählt, dass eine vollständige Abtastung des zweiten Bereichs nach x Segmenten erreicht ist, wobei x eine natürliche Zahl zwischen einschließlich 1 und der Anzahl der Segmente ist. Man kann also jedes Segment, immer nach zwei Segmenten, immer nach vier Segmenten, ..., oder nach der Messung aller Segmente einen vollständig abgetasteten zweiten Bereich 37 erhalten.

Im dritten Schritt S3 wird definiert, wann die Messsignale 36 und 43 des zweiten Bereichs 37 aufgenommen werden. Hier bestehen fünf Möglichkeiten. Entweder wird ein Teil der Messsignale 36 des zweiten Bereichs 37 im Rahmen des üblichen Messablaufs eines Segmentes 30, 31, 32, 33 aufgenommen und zusätzliche Messsignale 43 zu einem anderen Zeitpunkt. Dieser Zeitpunkt kann vor oder nach der Messung der Messsignale 35 und 36 des Segmentes liegen. Somit ergeben sich zwei Möglichkeiten.

Oder die zusätzlichen Messsignale 43 werden, als dritte Möglichkeit, während der Messung des zweiten Bereichs 37 eingeflochten. Dann können der zweite Bereich 37 vor oder nach oder zwischen der Messung des ersten Bereichs 34 und dritten Bereichs 38 akquiriert werden.

In zwei Fällen ist die Aufnahme der Messsignale 36 und 43 im zweiten Bereich 37 also unterbrochen, in drei Fällen ist sie direkt hintereinander.

Dabei wird immer das Vorgehen in einem Segment 30, 31, 32 oder 33 beschrieben. Die Aufnahme der Segmente 30, 31, 32 oder 33 erfolgt hintereinander.

In Schritt S4 wird ein Magnetresonanzdatensatz aufgenommen, wobei der k-Raum bzw. die Messsignale 35, 36 und 43 wie festgelegt abgetastet werden.

## Patentansprüche

1. Verfahren zur Aufnahme eines Magnetresonanzdatensatzes mit mehreren Spulen (6, 7, 8, 9), wobei die Messsignale (16, 35, 36, 43) des Magnetresonanzdatensatzes Messsignale (16, 35, 36, 43) aus wenigstens zwei Schichten (40, 41) enthalten, wobei die Messsignale (16, 35, 36, 43) segmentiert aufgenommen werden, **dadurch gekennzeichnet, dass** die Messsignale (16, 35, 36, 43) in einem ersten Bereich (34) des k-Raums mit einer ersten Abtastdichte $(\Delta k_{y1})$ und in einem zweiten Bereich (37) des k-Raums mit einer zweiten Abtastdichte $(\Delta k_{y2})$ aufgenommen werden.

2. Verfahren nach Anspruch 1, **dadurch gekennzeichnet, dass** die Messsignale in einem dritten Bereich (38) mit der ersten Abtastdichte $(\Delta k_{y1})$ aufgenommen werden.

3. Verfahren nach Anspruch 1 oder 2, **dadurch gekennzeichnet, dass** der zweite Bereich (37) die Mitte (42) des k-Raums umfasst.

4. Verfahren nach einem der vorangehenden Ansprüche, **dadurch gekennzeichnet, dass** die zweite Abtastdichte ($\Delta k_{y2}$) höher ist als die erste Abtastdichte ($\Delta k_{y1}$).

5. Verfahren nach einem der vorangehenden Ansprüche, **dadurch gekennzeichnet, dass** in jedem Segment (30, 31, 32, 33) die Messsignale (36) des zweiten Bereichs (37) vor den Messsignalen (35) des ersten Bereichs (34) und/oder vor den Messsignalen des dritten Bereichs (38) aufgenommen werden.

6. Verfahren nach einem der Ansprüche 1 bis 4, **dadurch gekennzeichnet, dass** in jedem Segment (30, 31, 32, 33) die Messsignale (36, 43) des zweiten Bereichs (37) nach den Messsignalen (35) des ersten Bereichs (34) und vor den Messsignalen des dritten Bereichs (38) aufgenommen werden.

7. Verfahren nach einem der Ansprüche 1 bis 4, **dadurch gekennzeichnet, dass** in jedem Segment (30, 31, 32, 33) die Messsignale (36, 43) des zweiten Bereichs (37) nach den Messsignalen (35) des ersten Bereichs (34) und nach den Messsignalen des dritten Bereichs (38) aufgenommen werden.

8. Verfahren nach einem der vorhergehenden Ansprüche, **dadurch gekennzeichnet, dass** in jedem Segment, (30, 31, 32, 33) ein Teil der Messsignale (36) des zweiten Bereichs (37) zwischen den Messsignalen (35) des ersten Bereichs (34) und den Messsignalen des dritten Bereichs (38) aufgenommen werden und ein Teil der Messsignale (43) des zweiten Bereichs (37) vor den Messsignalen (35) des ersten Bereichs (34) und/oder nach den Messsignalen des dritten Bereichs (38).

9. Verfahren nach einem der vorhergehenden Ansprüche, **dadurch gekennzeichnet, dass** die Messsignale (36, 42) des zweiten Bereichs (37) nach Durchlauf mindestens, insbesondere genau, zweier Segmente (30, 31, 32, 33) einen vollständigen Satz an Kalibrierungsdaten ergeben.

10. Verfahren nach einem der vorhergehenden Ansprüche, **dadurch gekennzeichnet, dass** die Messdatenaufnahme in wenigstens drei Segmente (30, 31, 32, 33) unterteilt ist.

11. Verfahren nach einem der vorhergehenden Ansprüche, **dadurch gekennzeichnet, dass** der k-Raum im zweiten Bereich (37) spätestens nach Durchlauf aller Segmente (30, 31, 32, 33) vollständig abgetastet ist.

12. Verfahren nach einem der vorhergehenden Ansprüche, **dadurch gekennzeichnet, dass** der k-Raum im zweiten Bereich (37) wenigstens zweimal vollständig abgetastet wird.

13. Computerprogrammprodukt (5) für eine Steuerungseinrichtung (3) zur Steuerung einer Datenerzeugungseinheit, insbesondere einer Bilderzeugungseinheit, einer Magnetresonanzanlage (1) zum Durchführen eines Verfahrens gemäß einem der vorhergehenden Ansprüche.

14. Datenträger (4) für eine Steuerungseinrichtung (3) zur Steuerung einer Datenerzeugungseinheit, insbesondere Bilderzeugungseinheit, einer Magnetresonanzanlage (1) mit Daten zum Durchführen eines Verfahrens gemäß einem der vorhergehenden Ansprüche.

15. Magnetresonanzanlage (1) mit einer Steuerungseinrichtung (3), **dadurch gekennzeichnet, dass** die Steuerungseinrichtung (3) zur Durchführung eines Verfahrens nach einem der Ansprüche 1 bis 12 ausgebildet ist.

**Geänderte Patentansprüche gemäss Regel 137(2) EPÜ.**

1. Verfahren zur Aufnahme eines Magnetresonanzdatensatzes mit mehreren Spulen (6, 7, 8, 9), wobei die Messsignale (16, 35, 36, 43) des Magnetresonanzdatensatzes Messsignale (16, 35, 36, 43) aus wenigstens zwei Schichten (40, 41) enthalten, wobei die Messsignale (16, 35, 36, 43) segmentiert aufgenommen werden, wobei der Magnetresonanzdatensatz mit mehreren Spulen parallel aufgenommen wird, in einer Anregungsphase ein Anregungsimpuls angelegt wird, der als Mehrbandimpuls ausgebildet ist zum gleichzeitigen Anregen mehrerer Schichten und in jedem Segment wenigstens ein Positionswechsel in Schichtauswahlrichtung durch einen Gradienten vollzogen wird, **dadurch gekennzeichnet, dass** die Messsignale (16, 35, 36, 43) in einem ersten Bereich (34) des k-Raums mit einer ersten Abtastdichte ($\Delta k_{y1}$) und in einem zweiten Bereich (37) des k-Raums mit einer zweiten Abtastdichte ($\Delta k_{y2}$) aufgenommen werden, wobei die Abtastdichte in $k_x$-Richtung immer gleich ist und die Abtastdichte in $k_y$- und/oder $k_z$-Richtung variiert wird.

2. Verfahren nach Anspruch 1, **dadurch gekennzeichnet, dass** die Messsignale in einem dritten Bereich (38) mit der ersten Abtastdichte ($\Delta k_{y1}$) aufgenommen werden.

3. Verfahren nach Anspruch 1 oder 2, **dadurch gekennzeichnet, dass** der zweite Bereich (37) die Mitte (42) des k-Raums umfasst.

**4.** Verfahren nach einem der vorangehenden Ansprüche, **dadurch gekennzeichnet, dass** die zweite Abtastdichte ($\Delta k_{y2}$) höher ist als die erste Abtastdichte ($\Delta k_{y1}$).

**5.** Verfahren nach einem der vorangehenden Ansprüche, **dadurch gekennzeichnet, dass** in jedem Segment (30, 31, 32, 33) die Messsignale (36) des zweiten Bereichs (37) vor den Messsignalen (35) des ersten Bereichs (34) und/oder vor den Messsignalen des dritten Bereichs (38) aufgenommen werden.

**6.** Verfahren nach einem der Ansprüche 1 bis 4, **dadurch gekennzeichnet, dass** in jedem Segment (30, 31, 32, 33) die Messsignale (36, 43) des zweiten Bereichs (37) nach den Messsignalen (35) des ersten Bereichs (34) und vor den Messsignalen des dritten Bereichs (38) aufgenommen werden.

**7.** Verfahren nach einem der Ansprüche 1 bis 4, **dadurch gekennzeichnet, dass** in jedem Segment (30, 31, 32, 33) die Messsignale (36, 43) des zweiten Bereichs (37) nach den Messsignalen (35) des ersten Bereichs (34) und nach den Messsignalen des dritten Bereichs (38) aufgenommen werden.

**8.** Verfahren nach einem der vorhergehenden Ansprüche, **dadurch gekennzeichnet, dass** in jedem Segment,(30, 31, 32, 33) ein Teil der Messsignale (36) des zweiten Bereichs (37) zwischen den Messsignalen (35) des ersten Bereichs (34) und den Messsignalen des dritten Bereichs (38) aufgenommen werden und ein Teil der Messsignale (43) des zweiten Bereichs (37) vor den Messsignalen (35) des ersten Bereichs (34) und/oder nach den Messsignalen des dritten Bereichs (38).

**9.** Verfahren nach einem der vorhergehenden Ansprüche, **dadurch gekennzeichnet, dass** die Messsignale (36, 42) des zweiten Bereichs (37) nach Durchlauf mindestens, insbesondere genau, zweier Segmente (30, 31, 32, 33) einen vollständigen Satz an Kalibrierungsdaten ergeben.

**10.** Verfahren nach einem der vorhergehenden Ansprüche, **dadurch gekennzeichnet, dass** die Messdatenaufnahme in wenigstens drei Segmente (30, 31, 32, 33) unterteilt ist.

**11.** Verfahren nach einem der vorhergehenden Ansprüche, **dadurch gekennzeichnet, dass** der k-Raum im zweiten Bereich (37) spätestens nach Durchlauf aller Segmente (30, 31, 32, 33) vollständig im Hinblick auf das Nyquist-Theorem abgetastet ist.

**12.** Verfahren nach einem der vorhergehenden Ansprüche, **dadurch gekennzeichnet, dass** der k-Raum im zweiten Bereich (37) wenigstens zweimal vollständig im Hinblick auf das Nyquist-Theorem abgetastet wird.

**13.** Computerprogrammprodukt (5) für eine Steuerungseinrichtung (3) zur Steuerung einer Datenerzeugungseinheit, insbesondere einer Bilderzeugungseinheit, einer Magnetresonanzanlage (1) zum Durchführen eines Verfahrens gemäß einem der vorhergehenden Ansprüche.

**14.** Datenträger (4) für eine Steuerungseinrichtung (3) zur Steuerung einer Datenerzeugungseinheit, insbesondere Bilderzeugungseinheit, einer Magnetresonanzanlage (1) mit Daten zum Durchführen eines Verfahrens gemäß einem der vorhergehenden Ansprüche.

**15.** Magnetresonanzanlage (1) mit mehreren Spulen und einer Steuerungseinrichtung (3), **dadurch gekennzeichnet, dass** die Steuerungseinrichtung (3) zur Durchführung eines Verfahrens nach einem der Ansprüche 1 bis 12 ausgebildet ist.

FIG 1

EP 3 561 535 A1

FIG 2

FIG 3

FIG 4

EP 3 561 535 A1

FIG 5

EP 3 561 535 A1

# FIG 6

S1

S2

S3

S4

Europäisches
Patentamt

European
Patent Office

Office européen
des brevets

**EUROPÄISCHER RECHERCHENBERICHT**

Nummer der Anmeldung

EP 18 16 9552

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | STEPHEN R. YUTZY ET AL: "Improvements in multislice parallel imaging using radial CAIPIRINHA", MAGNETIC RESONANCE IN MEDICINE, Bd. 65, Nr. 6, 1. Februar 2011 (2011-02-01), Seiten 1630-1637, XP055133595, ISSN: 0740-3194, DOI: 10.1002/mrm.22752 * Abschnitt "THEORY" * * Abschnitt "MATERIALS AND METHODS" * * Abbildungen 1,2 * ----- | 1-7,9-15 | INV. G01R33/48 G01R33/483 G01R33/561 |
| X | MENGYE LYU ET AL.: "Multi-band PROPELLER Imaging with Auto-calibration", PROCEEDINGS OF THE INTERNATIONAL SOCIETY FOR MAGNETIC RESONANCE IN MEDICINE, ISMRM, 23RD ANNUAL MEETING AND EXHIBITION, TORONTO, ONTARIO, CANADA, 30 MAY - 5 JUNE 2015, Nr. 2411, 15. Mai 2015 (2015-05-15), XP040668088, * das ganze Dokument * ----- | 1-15 | |
| X | KANGRONG ZHU ET AL.: "Autocalibrating CAIPIRINHA: Reformulating CAIPIRINHA as a 3D Problem", PROCEEDINGS OF THE INTERNATIONAL SOCIETY FOR MAGNETIC RESONANCE IN MEDICINE, ISMRM, 20TH ANNUAL MEETING AND EXHIBITION, MELBOURNE, AUSTRALIA, 5-11 MAY 2012, 21. April 2012 (2012-04-21), XP040622948, * das ganze Dokument * ----- -/-- | 1-4,9-15 | RECHERCHIERTE SACHGEBIETE (IPC) G01R |

Der vorliegende Recherchenbericht wurde für alle Patentansprüche erstellt

| Recherchenort | Abschlußdatum der Recherche | Prüfer |
|---|---|---|
| München | 13. November 2018 | Durst, Markus |

EPO FORM 1503 03.82 (P04C03)

Seite 1 von 2

Europäisches Patentamt
European Patent Office
Office européen des brevets

## EUROPÄISCHER RECHERCHENBERICHT

Nummer der Anmeldung

EP 18 16 9552

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | MARIO ZELLER ET AL: "Boosting BOLD fMRI by K-Space Density Weighted Echo Planar Imaging", PLOS ONE, Bd. 8, Nr. 9, 10. September 2013 (2013-09-10), Seite e74501, XP055522937, DOI: 10.1371/journal.pone.0074501 * Abbildung 1 * * Abschnitt "Methods" * * Abschnitt "Discussion and Conclusion" * ----- | 1-15 | |
| A | ERPENG DAI ET AL: "Simultaneous multislice accelerated interleaved EPI DWI using generalized blipped-CAIPI acquisition and 3D K-space reconstruction : SMS Accelerated iEPI DWI", MAGNETIC RESONANCE IN MEDICINE., Bd. 77, Nr. 4, 19. April 2016 (2016-04-19), Seiten 1593-1605, XP055493908, US ISSN: 0740-3194, DOI: 10.1002/mrm.26249 * Abbildungen 1,2 * * Abschnitt "METHODS" * ----- | 1-15 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 13. November 2018 | Durst, Markus |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.....................................................................
& : Mitglied der gleichen Patentfamilie, übereinstimmendes Dokument

EPO FORM 1503 03.82 (P04C03)

Seite 2 von 2

EP 3 561 535 A1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **BREUER F.A. ; BLAIMER M. ; HEIDEMANN R.M. ; MÜLLER M.F. ; GRISWOLD M.A. ; JAKOB P.M.** Controlled Aliasing In Parallel Imaging Results IN Higher Acceleration for Multi-Slice Imaging. *Magn. Res. Med.,* vol. 53, 684-691

- **SETSOMPOP K. ; GAGOSKI B.A. ; POLIMENI J.R. ; WITZEL T. ; VAN WEDEEN J. ; WALD L.L.** Blipped-Controlled Aliasing in Parallel Imaging (blipped-CAIPI) for simultaneous multi-slice EPI with reduced g-factor penalty. *Magn. Res. Med.,* vol. 67, 1210-1224